# EUROPÄISCHE PATENTSCHRIFT

(11) **EP 0 982 296 B1**
(45) Veröffentlichungstag und Bekanntmachung des Hinweises auf die Patenterteilung: **18.12.2002**
(21) Anmeldenummer: 99115239.8
(22) Anmeldetag: 02.08.1999
(51) Int. Cl.: C07C 323/22, C07C 323/12, C07D 317/20, C07D 319/08, C07C 319/04, A23L 1/226, A61K 7/46

(54) **Duft- und Aromastoff**
Perfuming and flavouring agents
Agents parfumants et aromatisants

(30) Priorität: 20.08.1998 DE 19837702
(43) Veröffentlichungstag der Anmeldung: 01.03.2000
(73) Patentinhaber: Dragoco Gerberding & Co Aktiengesellschaft, 37603 Holzminden (DE)
(72) Erfinder: Sabater-Lüntzel, Christopher, Dr., 37671 Höxter-Ottbergen (DE); Widder, Sabine, Dr., 37603 Holzminden (DE); Pickenhagen, Wilhelm, Dr., 37671 Höxter (DE)
(74) Vertreter: Eisenführ, Speiser & Partner

(56) Entgegenhaltungen:
- EP-A- 0 924 198
- DE-A- 2 338 680
- US-A- 4 496 600
- P. WERKHOFF ET AL: ADV. FOOD SCI., Bd. 18, Nr. 1/2, 1996, Seiten 19-27, XP002125411
- H.W. SCHNABEL ET AL: JUSTUS LIEBIGS ANN. CHEM., Nr. 3, 1974, Seiten 477-495, XP002125412

## Beschreibung

Die Erfindung betrifft einen neuen Duft- und Aromastoff sowie ein Verfahren zur Herstellung dieses neuen Stoffes.

Lebensmittel werden in der heutigen Zeit regelmäßig aromatisiert. Die Mehrheit der Konsumenten in den modernen Industriegesellschaften erwartet nämlich eine breite Palette schmackhafter Lebensmittel zu erschwinglichen Preisen. Die Schmackhaftigkeit von Lebensmitteln ist von großer Bedeutung, da sie in der Regel eine gute Bekömmlichkeit bewirkt. Die Aromenindustrie stellt bereits eine Vielzahl von Aromen zur Verfügung, um Lebensmittel einer breiten Bevölkerungsschicht verfügbar und schmackhaft zu machen.

Aromastoffe werden eingesetzt, um (a) Lebensmittelprodukten ohne Eigengeschmack eine Geschmacksnote zu verleihen oder um (b) Aromaverluste auszugleichen, die beispielsweise im Rahmen des Herstellungsprozesses eines Lebensmittels auftreten.

Die DE 23 38 680 A (Firmenich) offenbart aliphatische und cycloaliphatische Merkaptoderivate, Verfahren zu deren Herstellung und deren Verwendung als Mittel zur Verbesserung, Verstärkung oder Veränderung der organoleptischen Eigenschaften von Nahrungs- und Futtermitteln, Getränken, pharmazeutischen Präparaten und Tabakprodukten.

Aus der US-A-4,496,600 (Parliment) sind mercaptoalkyl-substituierte Oxathiolane und Oxathiane bekannt, welche als Geschmacksstoffzusammensetzung für Nahrungsmittel eingesetzt werden können.

Aus Adv. Food Sci. Vol. 18 No. 1/2, 1996, S. 19-27 sind bestimmte mercapto/methylthiosubstituierte Aldehyde und Ketone bekannt, die in gekochter Rindsleber enthalten sind.

Aus Liebigs Ann. Chem. 1974, S. 477-495 ist eine Synthese von Cephamderivaten bekannt, und bestimmte Thiole sind offenbart.

Der vorliegenden Erfindung lag die Aufgabe zugrunde, einen neuen Stoff zur Aromatisierung von Lebensmitteln (nachfolgend auch kurz "Aromastoff" genannt) anzugeben, und zwar insbesondere einen Stoff mit einem Fleischaroma.

Erfindungsgemäß werden die nachfolgenden Verbindungen der allgemeinen Formel A 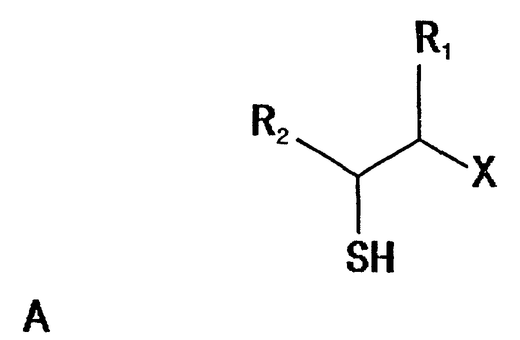 mit X = CHO, 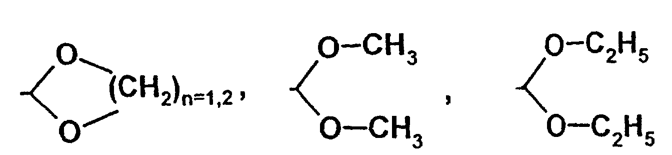 R₁ = CH₃, C₂H₅, n-C₃H₇, iso -C₃H₇, n-C₄H₉, iso-C₄H₉ oder tert-C₄H₉ und
R₂ = CH₃, C₂H₅, n-C₃H_{7,} iso-C₃H₇, n-C₄H₉, iso-C₄H₉ oder tert-C₄H₉
als Aromastoff angegeben. überraschenderweise hat sich nämlich gezeigt, daß diese neu synthetisierten Verbindungen, Aldehyde, z.B. 3-Mercapto-2-methylpentanal, und Acetale, bereits in niedrigen Konzentrationen hervorragend dazu geeignet sind, Lebensmitteln, insbesondere Fleischbrühen und anderen Fleischspeisen eine interessante geruchliche und geschmackliche Note zu verleihen. Auf Grund der sehr hohen geruchlichen und geschmacklichen Intensität der erfindungsgemäßen Verbindungen können sie in großer Verdünnung eingesetzt werden; die genauen Konzentrationen oder Mengen zur Aromatisierung eines Lebensmittels wird der Fachmann auf übliche Weise an die jeweiligen Wünsche und Bedürfnisse des Einzelfalls anpassen.

Die erfindungsgemäßen Verbindungen sind überdies auch zur Verwendung als Duftstoff geeignet, und zwar insbesondere als Duftstoff in der Parfümindustrie; sie haben generell einen besonders niedrigen Geruchsschwellenwert, was sich als vorteilhaft erweist, da bereits geringe Mengen einer erfindungsgemäßen Verbindung zur Erzeugung eines erwünschten Geruches ausreichen.

Den erfindungsgemäßen Verbindungen gemeinsam sind folgende strukturelle Merkmale, die überraschenderweise nur bei gleichzeitiger Anwesenheit in einem Molekül die erwünschten Eigenschaften erzeugen:
- eine Aldehyd- oder Acetal-Funktion am C-1,
- eine Mercapto-Gruppe (SH-Gruppe) am C-3 und
- ein Alkylrest am C-2, d.h. eine Kettenverzweigung.

Die Erfindung betrifft neben den erfindungsgemäßen Verbindungen auch Verfahren zu ihrer Herstellung.

Bei einem erfindungsgemäßen Verfahren zur Herstellung eines 3-Mercapto-2-alkyl-alkanals der Formel A mit X = CHO, R₁ = CH₃, C₂H₅, n-C₃H₇, iso-C₃H₇, n-C₄H₉, iso-C₄-H₉ oder tert-C₄H₉ und R₂ = CH₃, C₂H₅, n-C₃H₇, iso-C₃H₇, n-C₄H₉, iso-C₄H₉ oder tert-C₄H₉ wird das korrespondierende 2-Alkyl-2-alkenal mit Schwefelwasserstoff umgesetzt.

Und schließlich betrifft die Erfindung noch aromatisierte Lebensmittel mit einem Anteil an einer oder mehreren erfindungsgemäßen Verbindungen sowie die Verwendung der erfindungsgemäßen Verbindungen als 'Duft- oder Aromastoff.

Die erfindungsgemäßen Aldehyde, z.B. 3-Mercapto-2-methyl-pentanal, sind sensorisch höchst interessant. Sie besitzen aber noch weitere Eigenschaften, die über ihre rein geruchlichen und geschmacklichen Eigenschaften weit hinausgehen.

So haften die erfindungsgemäßen Aldehyde, bei denen es sich ja um Substanzen vergleichsweise geringer Größe handelt, auf unterschiedlichen Oberflächen - auch im Mund - überraschend lange an. Diese gute Anhaftung (das sogenannte "long lasting") zeigt sich z.B. auf einem üblichen Riechstreifen. Noch Tage nach dem Aufbringen einer niedrig konzentrierten Lösung eines erfindungsgemäßen Aldehyds, z.B. 3-Mercapto-2-methyl-pentanals auf den Riechstreifen kann der besondere Geruch des Aldehyds ohne Mühe wahrgenommen werden. Diese Eigenschaft ist insbesondere für die Parfümindustrie von Bedeutung, denn hier besteht ein ständiger Bedarf an neuen und interessanten Verbindungen, die lange anhaften.

Ein weiterer überraschender Effekt, der bei der Verwendung der erfindungsgemäßen Aldehyde auftritt, ist das sogenannte "Mouthfeel". Insbesondere bei einer Verwendung der erfindungsgemäßen Aldehyde in Fleischaroma-Kompositionen tragen sie dazu bei, die Fülle, das Volumen und den Körper des Fleischaromas zu verstärken. "Mouthfeel"-Eigenschaften treten normalerweise nur bei höhermolekularen Substanzen auf, wie z.B. bei Pektinen oder Stärkeprodukten.

Anstelle des direkten Einsatzes der erfindungsgemäßen Aldehyde als Duftoder Aromastoff oder als "Mouthfeel"-Vermittler ist es manchmal erwünscht, chemische Derivate einzusetzen, die sich unter den Bedingungen einer üblichen Weiterverarbeitung - z.B. im sauren Milieu, beim Kochen oder Braten, an der Luft oder dergleichen - in den jeweiligen Aldehyd umwandeln oder umwandeln lassen. Die Derivate können dabei selbst sensorisch interessant sein, so wie die erfindungsgemäßen Acetale, müssen es aber nicht.

Nachfolgend wird die Erfindung anhand von Beispielen näher erläutert:

### Beispiel 1: Herstellung von 3-Mercapto-2-methyl-pentanal

In einem auf 40°C temperierten 500ml-Rührwerk mit Gaseinleitungsrohr werden 321g frisch destilliertes 2-Methyl-2-pentenal, 23g Triethylamin und 1,8g Hydrochinon in 600ml trockenem Tetrahydrofuran gelöst. Bei 40°C wird Schwefelwasserstoff über ca. 6h in einem kräftigen Strom eingeleitet. Der entweichende Schwefelwasserstoff wird durch eine nachgeschaltete Waschflasche mit Natronlauge gebunden. Nach der Reaktion wird für 5min Stickstoff durch die Reaktionslösung geleitet, um überschüssigen Schwefelwasserstoff auszutreiben. Zur Isolierung des 3-Mercapto-2-methyl-pentanals wird die Reaktionslösung mit Wasser und gesättigter Kochsalzlösung gewaschen, über Natriumsulfat getrocknet und im Teilvakuum vom Lösungsmittel befreit.

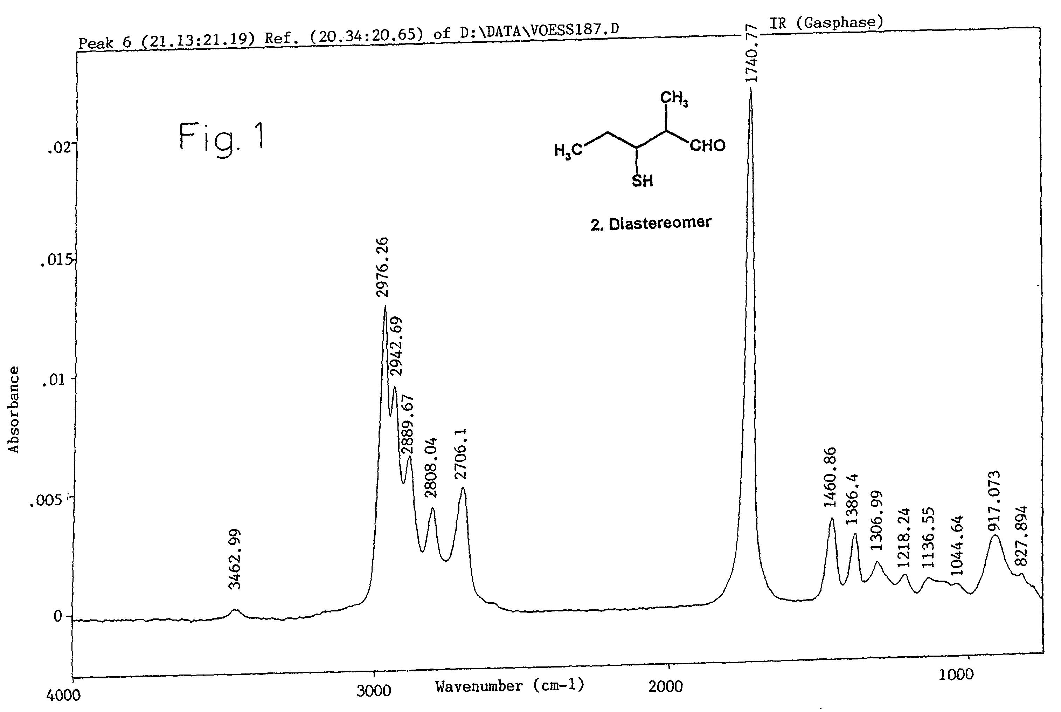

Bei der GC/MS-Analyse des Rückstandes erhält man beide diastereomeren Mercaptoaldehyde im Verhältnis 1:1, wie sich aus den beigefügten Spektren (Figuren 1-4) ergibt.
- Anmerkung:: Das in Beispiel 1 erwähnte 2-Methyl-2-pentenal ist kommerziell erhältlich. Weitere ungesättigte Precursor-Aldehyde, die ebenfalls zu erfindungsgemäßen Aldehyden umgesetzt wurden aber hier nicht individuell genannt werden, sind entweder ebenfalls kommerziell erhältlich (z.B. 2,3-Dimethylacrolein) oder leicht durch bekannte Syntheseverfahren zugänglich (α,β-ungesättigte Aldehyde durch gezielte Aldolkondensation: L. Brandsma, Preparative Polar Organometallic Chemistry 2, p. 145, Springer Verlag, Heidelberg 1990).

### Beispiel 2: Herstellung der 3-Mercapto-2-alkyl-alkanal-acetale (insbesondere der 3-Mercapto-2-methyl-pentanal-acetale) am Beispiel von 2-(1,3-dioxolan-2-yl)-pentan-3-thiol

### 2.1. Umsetzung von 2-Methyl-2-pentenal mit Thioessigsäure zu 1-Ethyl-2-methyl-3-oxopropyl-thioacetat

In einer Rührapparatur werden 9,8g (0,1mol) 2-Methyl-2-pentenal vorgelegt und bei 0°C unter Stickstoffatmosphäre zunächst 100mg Piperidin und anschließend langsam 11,4g (0,15mol) Thioessigsäure zugetropft. Anschließend läßt man die Reaktionsmischung auf Raumtemperatur erwärmen und über Nacht weiterrühren. Das Reaktionsgemisch wird dann mit 100ml Diethylether verdünnt, zunächst mit 20ml 2N Salzsäure und danach zweimal mit je 30ml 5%iger Natriumhydrogencarbonatlösung gewaschen. Anschließend wird die organische Phase über Natriumsulfat getrocknet, filtriert und das Lösungsmittel im Teilvakuum vorsichtig abgezogen. Eine weitere Aufreinigung ist für den nächsten Reaktionsschritt nicht notwendig.

### 1-Ethyl-2-methyl-3-oxopropyl-thioacetat

**MS** (EI, 70eV): 174 (M⁺ 0,5), 131 (17), 103 (12), 70 (32), 69 (12), 61 (11), 43 (100), 41 (20)

### 2.2. Acetalisierung von 1-Ethyl-2-methyl-3-oxopropyl-thioacetat mit Ethylenglycol

10g (57mmol) der oben dargestellten Verbindung werden mit 7,01g (114mmol) Ethylengylcol, 150mg p-Toluolsulfonsäure sowie 300ml Toluol 3 Stunden unter Rückfluß am Wasserabscheider gekocht.

Anschließend wird die Reaktionsmischung mit zweimal 40ml gesättigter Natriumhydrogencarbonatlösung gewaschen, über Natriumsulfat getrocknet, filtriert und das Lösungsmittel im Teilvakuum vorsichtig abgezogen. Eine weitere Aufreinigung ist für den nächsten Reaktionsschritt nicht notwendig.

### 2-(1,3-dioxolan-2-yl)-1-etyhlpropyl-thioacetat

**MS** (EI, 70eV): kein M⁺, 175 (1), 148 (12), 101 (4), 74 (4), 73 (100), 45 (15), 43 (17)

### 2.3. Hydrolyse von 2-(1,3-dioxolan-2-yl)-1-ethylpropyl-thioacetat

In einer Rührapparatur werden zu 115ml 0,5N Natriumhydroxidlösung sowie 200ml Methanol bei Raumtemperatur langsam 5g (23mmol) 2-(1,3-dioxolan-2-yl)-1-ethlpropyl-thioacetat zugetropft. Anschließend läßt man die Reaktionsmischung bei Raumtemperatur weitere 18 Stunden rühren.

Nach beendeter Reaktion wird das Methanol am Teilvakuum entfernt und Nebenprodukte durch Extraktion der wässrigen Lösung mit 100ml Diethylether entfernt. Anschließend wir mit 2N Salzsäure auf pH 1 angesäuert und das Mercaptoacetal mit Diethylether (4 x 50ml) extrahiert. Die vereinigten organischen Phasen werden mit gesättigter 30ml Natriumhydrogencarbonatlösung gewaschen, über Natriumsulfat getrocknet, filtriert und das Lösungsmittel vorsichtig im Teilvakuum abgezogen. Das 2-(1,3-dioxolan-2-yl)-pentan-3-thiol liegt in einer Reinheit von >95% (GC) vor.

### 2-(1,3-dioxolan-2-yl)-pentan-3-thiol

**MS** (EI, 70eV): 176 (M⁺ 0,3), 143 (6), 113 (5), 74 (4), 73 (100), 45 (20), 41 (8)

### Beispiel 3: Sensorische Untersuchung einer ausgewählten erfindungsgemäßen Verbindung

### 3-Mercapto-2-methylpentanal

| | | |
|---|---|---|
| 3.1. | Dosierung | 0,1 ppm |
| | Geruch | fleischig |
| | Geschmack | fleischig, an Fleischbrühe erinnernd, an gekochtes Fleisch erinnernd. |
| 3.2. | Geruchsschwellenwert | sehr niedrig |

### Beispiel 4: Spektroskopische Daten einer ausgewählten erfindungsgemäßen Verbindung

### 3-Mercapto-2-methylpentanal

**FTIR** (gas-phase): Diastereomer 1: 2977 (m), 2942 (m), 2890 (m), 2809 (w), 2708 (m), 1739 (s), 1460 (w), 1387 (w), 1305 (w); Diastereomer 2: 2976 (m), 2942 (m), 2890 (m), 2808 (w), 2706 (m), 1740 (s), 1461 (w), 1386 (w), 1307 (w)

w=schwache, m=mittelstarke, s=starke Bande

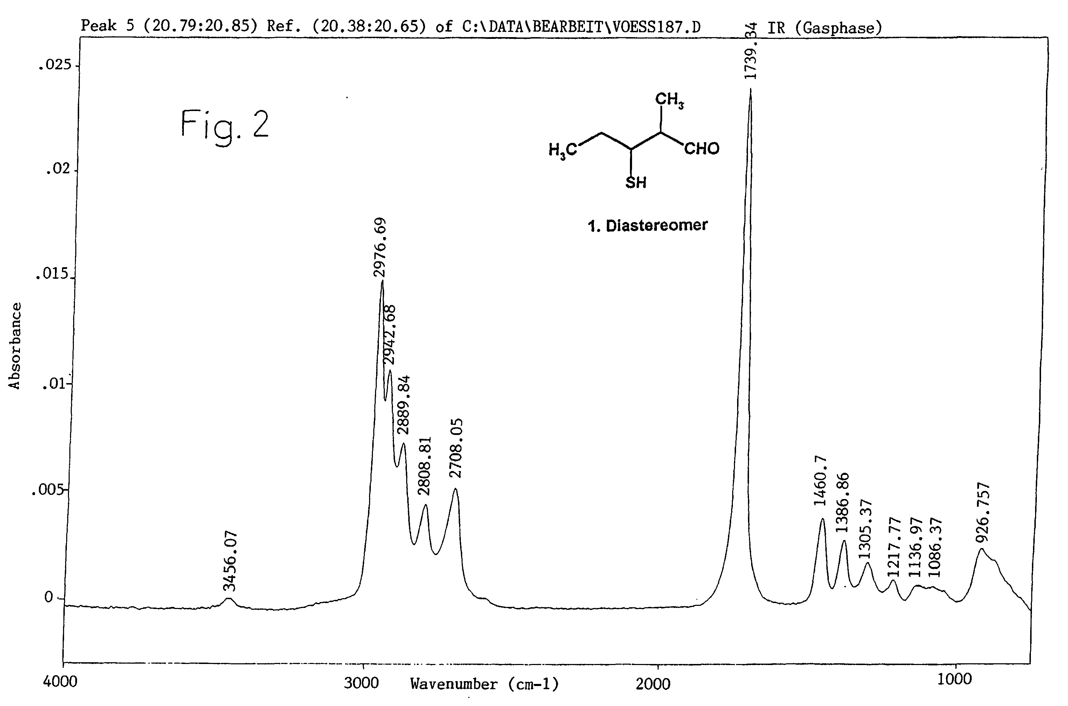

Diesen Daten entsprechen die Spektren gemäß den Figuren 1 und 2.

**MS** (EI, 70 eV): Diastereomer 1: 132 (M⁺, 12), 114 (13), 99 (30), 75 (33), 70 (100), 61 (24), 55 (82), 43 (39), 41 (97); Diastereomer 2: 132 (M⁺, 13) 114 (18); 99 (41), 75 (39), 70 (75), 61 (24), 55 (67), 43 (43), 41 (100)

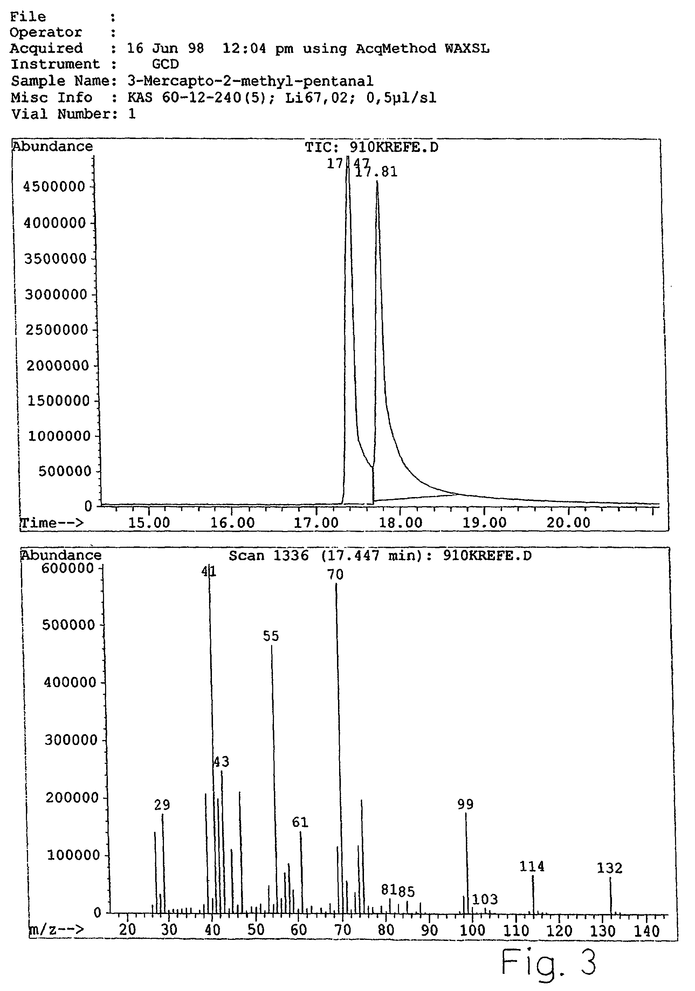

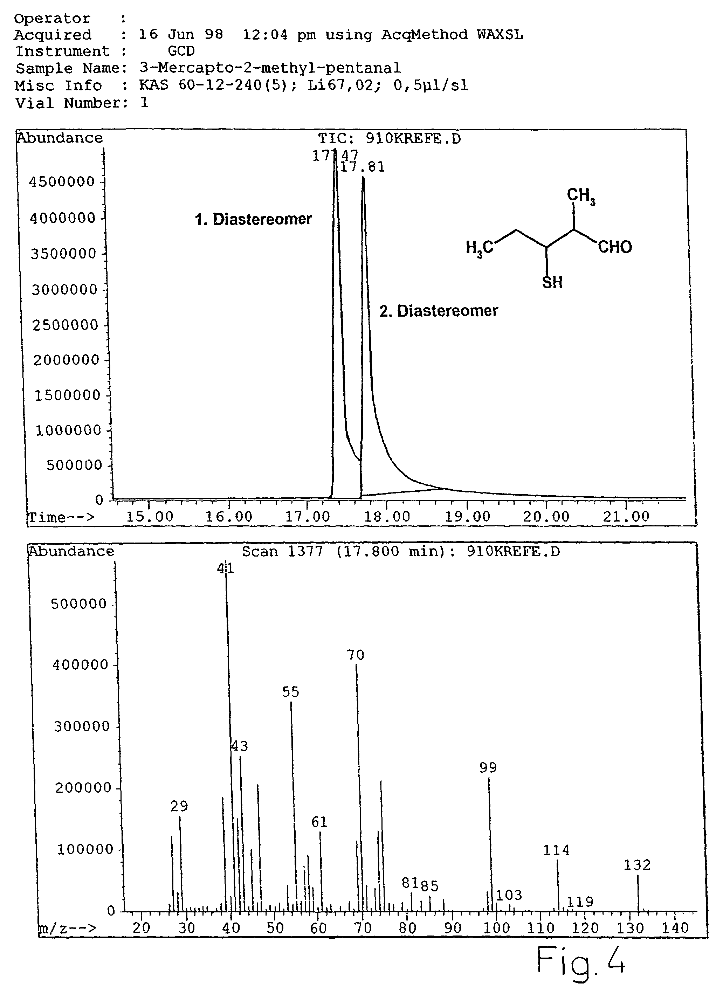

Diesen Daten entsprechen die Spektren gemäß den Figuren 3 und 4.

## Patentansprüche

1. Verbindung der allgemeinen Formel A, 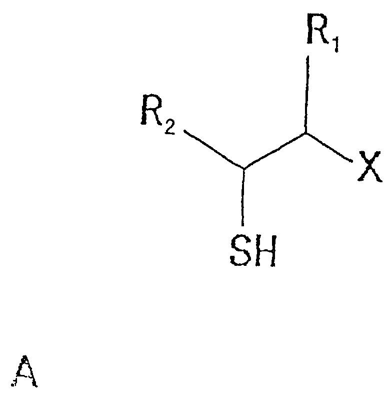 mit X = CHO, 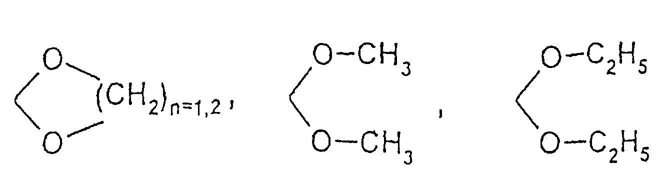 R₁ = CH₃, C₂H₅, n-C₃H₇, iso-C₃H₇, n-C₄H₉, iso-C₄H₉ oder tert.-C₄H₉ und
R₂ = CH₃, C₂H₅, n-C₃H₇, iso-C₃H₇, n-C₄H₉, iso-C₄H₉ oder tert.-C₄H₉.

2. Verfahren zur Herstellung eines 3-Mercapto-2-alkyl-alkanals der allgemeinen Formel A mit X = CHO, in dem das korrespondierende 2-Alkyl-2-alkenal mit Schwefelwasserstoff umgesetzt wird.

3. Verwendung einer Verbindung gemäß Anspruch 1 mit X =CHO als Duft- oder Aromastoff.

4. Duft- oder Aromastoffformulierung, umfassend zumindest eine Verbindung gemäß Anspruch 1 mit X = CHO.

5. Aromatisiertes Lebensmittel mit einem Anteil an einer Verbindung gemäß Anspruch 1.

## Claims

1. A compound of the general formula A 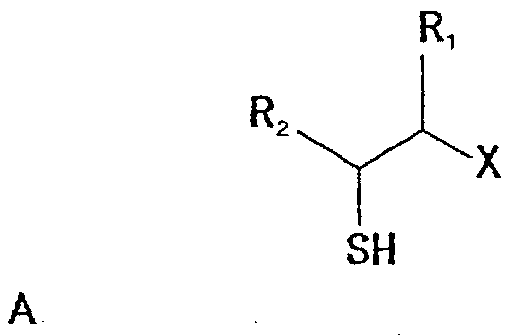 where
X = CHO, 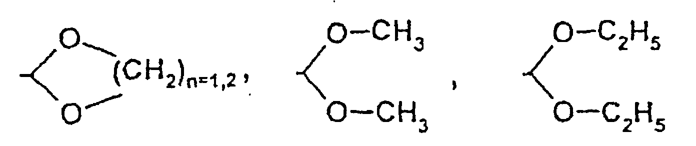 R₁ = CH₃, C₂H₅, n-C₃H₇, iso-C₃H₇, n-C₄H₉, iso-C₄H₉ or tert.-C₄H₉ and
R₂ = CH₃, C₂H₅, n-C₃H₇, iso-C₃H₇, n-C₄H₉, iso-C₄H₉ or tert.-C₄H₉.

2. A process for the production of a 3-mercapto-2-alkyl-alkanal of the general formula A where X = CHO, in which the corresponding 2-alkyl-2-alkenal is reacted with hydrogen sulfide.

3. Use of a compound according to claim 1 where X = CHO as a fragrance or flavour material.

4. A fragrance or flavour formulation comprising at least one compound according to claim 1 where X = CHO.

5. A flavoured foodstuff with a content of a compound according to claim 1.

## Revendications

1. Composé de la formule générale A : 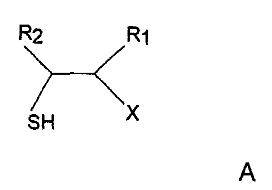 avec X = CHO, 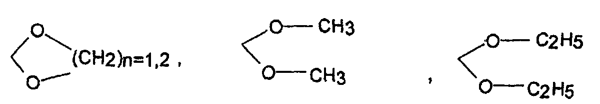 R₁ = CH₃, C₂H₅, n-C₃H₇, i-C₃H₇, n-C₄H₉, i-C₄H₉ ou t-C₄H₉, et
R₂ = CH₃, C₂H₅, n-C₃H₇, i-C₃H₇, n-C₄H₉, i-C₄H₉ ou t-C₄H₉.

2. Procédé de préparation d'un 3-mercapto-2-alkylalcanal de la formule générale A, avec X = CHO, dans lequel le 2-alkyl-2-alcènal correspondant est mis à réagir avec du sulfure d'hydrogène.

3. Utilisation d'un composé selon la revendication 1 avec X = CHO, comme agents parfumants et aromatisants.

4. Formulation parfumante et aromatisante, comprenant au moins un composé selon la revendication 1 avec X = CHO.

5. Aliment aromatisé avec une quantité en un composé selon la revendication 1.
